# EUROPÄISCHE PATENTANMELDUNG

(11) **EP 3 470 113 A1**
(43) Veröffentlichungstag der Anmeldung: **17.04.2019**
(21) Anmeldenummer: 17195924.0
(22) Anmeldetag: 11.10.2017
(51) Int. Cl.: A61N 1/372

(54) **ANTENNE EINES IMPLANTAT-PROGRAMMIERGERÄTES UND PROGRAMMIERGERÄT**

(71) Anmelder: BIOTRONIK SE & Co. KG, 12359 Berlin (DE)
(72) Erfinder: Hammerschmidt, Christoph, 12435 Berlin (DE); Merlin, Julian, 10713 Berlin (DE); Freixas Montolio, Joaquim, 10961 Berlin (DE); Stephan, Enrico, 10707 Berlin (DE); Krips, Oliver, 13437 Berlin (DE); Fechner, Andreas, 10243 Berlin (DE); Grund, Peter, 13353 Berlin (DE)
(74) Vertreter: Keck, Hans-Georg

(57) **Zusammenfassung**

Antenne eines Implantat-Programmiergerätes, das eine, insbesondere im ULP-AMI-Band arbeitende, Telemetrie-Sende-/Empfangsstufe zur drahtlosen Kommunikation mit einem medizinelektronischen Implantat aufweist, wobei die Antenne als Leiterzug auf einem steifen flächigen Trägersubstrat ausgebildet ist, dessen Fläche um ein Vielfaches größer als die vom Leiterzug belegte Fläche ist.

## Beschreibung

Die Erfindung betrifft eine Antenne eines Implantat-Programmiergerätes, das eine, insbesondere im 401-406MHz (ULP-AMI, MICS, MEDS)-Band arbeitende, Telemetrie-Sende-/Empfangsstufe zur drahtlosen Kommunikation mit einem medizinelektronischen Implantat aufweist. Sie betrifft des Weiteren ein entsprechendes Programmiergerät als solches.

Medizinelektronische Implantate, wie etwa Herzschrittmacher, implantierbare Kardioverter, Cochlearimplantate, implantierbare Medikamentendosierpumpen oder Neurostimulatoren, sind mit einer eingebauten Betriebssteuereinheit versehen und arbeiten weitgehend autonom aufgrund eines dort als Hardware implementierten oder in einem Arbeitsspeicher als Software abgelegten Steuerprogramms. In vielen Ausführungen und Anwendungsbereichen ist jedoch außerdem der Einsatz eines externen Steuer- bzw. Programmiergerätes und/oder einer Übertragung von im Körper des Patienten aufgenommenen Signalen nach außerhalb und beispielsweise auch eine Übermittlung des Batterie-Ladezustandes vorgesehen. Hierzu dienen spezielle Zusatzgeräte, die nachfolgend auch als "Implantat-Programmiergeräte" bezeichnet werden, obwohl ihre Funktion nicht auf die Aufgabe einer Umprogrammierung bzw. Betriebsprogramm-Aktualisierung von Implantaten beschränkt ist.

Derartige Programmiergeräte sind mit dem medizinelektronischen Implantat üblicherweise temporär über eine sogenannte Telemetrie-Übertragungsstrecke, also eine kurzreichweitige drahtlose Signalübertragungsstrecke auf Basis amplituden- oder frequenzmodulierter elektromagnetischer Wellen, verbunden.

Es versteht sich, dass für eine solche Signalübertragung eine Funkantenne benötigt wird. Diese ist üblicherweise im Programmiergerät eingebaut und hat bei bekannten Programmiergeräten die Form gebogener Drähte, die über Befestigungsplatinen und Schraubverbindungen mechanisch fixiert und speziell mit einem Gegenpol (etwa einem EMV-Schirmblech) verbunden sind. Eine Form-Fixierung kann dabei durch Klemmen im Gerätegehäuse erfolgen.

Bei bekannten Antennenkonstruktionen haben sich bestimmte Nachteile gezeigt:
Drahtantennen sind sehr empfindlich gegenüber mechanischen Deformationen. Der Abstand zwischen EMV-Schirmblech des Programmiergeräte-Gehäuses und dem offenen Antennenende kann vom definierten Abstand abweichen. Dadurch wird die Antenne aus dem Sende-/Empfangs-Band verstimmt. Dies führt direkt zur Verschlechterung der Signalqualität der Funkverbindung zum Implantat.

Der Erfindung liegt daher die Aufgabe zugrunde, eine hinsichtlich ihrer Funktion, insbesondere ihrer Unempfindlichkeit gegenüber mechanischen Einflüssen, verbesserter Antenne der gattungsgemäßen Art anzugeben. Weiterhin soll ein entsprechend verbessertes Programmiergerät angegeben werden.

Diese Aufgabe wird durch eine Antenne mit den Merkmalen des Anspruchs 1 und nachgeordnet durch ein Programmiergerät mit den Merkmalen des Anspruchs 11 gelöst. Zweckmäßige Fortbildungen des Erfindungsgedankens sind Gegenstand der abhängigen Ansprüche.

Die Erfindung schließt den Gedanken einer bewussten Abkehr vom üblichen Antennenaufbau aus selbsttragenden Leiterdrähten ein. Sie schließt weiter das Vorsehen eines flächigen Trägersubstrats für die Antenne und schließlich den Gedanken einer Ausführung der eigentlichen Antenne als Leiterzug auf einem solchen Substrat ein. Im Interesse einer hohen mechanischen Unempfindlichkeit ist das Trägersubstrat im Wesentlichen steif und seine Fläche um ein vielfaches größer als die vom Leiterzug belegte Fläche.

In einer Ausführung der Erfindung besteht das Trägersubstrat aus einem Leiterplattenmaterial. Insbesondere kann es aus einem Epoxidharz-Glasfasergewebe-Verbundmaterial, insbesondere vom FR4-Typ, bestehen. Alternativ kann es aus einem Material auf Hartpapierbasis, insbesondere vom FR2-Typ, bestehen.

Noch weiterhin ist vorgesehen, dass das Trägersubstrat aus einer Keramik besteht, die insbesondere Aluminiumoxide, Aluminiumnitrit oder LTCC aufweist. In einer noch weiteren Ausführung besteht das Trägersubstrat aus einem PTFE-Material.

In einer weiteren Ausführung der Erfindung ist benachbart zu einem Abschnitt des Leiterzuges mindestens eine Ausnehmung oder lokale Ausdünnung im Trägersubstrat vorgesehen. Durch diese Ausführung werden insbesondere mögliche Beeinträchtigungen der Antenneneigenschaften durch das Trägermaterial minimiert. Insbesondere ist die Ausnehmung oder lokale Ausdünnung nahe dem freien Ende des Leiterzuges vorgesehen.

In einer weiteren Ausführung der Erfindung ist die Antenne mit Anpassungsmitteln zur Anpassung an die Telemetrie-Sende-/Empfangsstufe oder deren Antennenanschluss versehen, welche auf dem Trägersubstrat integriert sind. Dies vereinfacht die Herstellung der Gesamtbaugruppe aus Antenne und Anpassungsmitteln und trägt zu niedrigen Herstellungskosten bei.

In einer aus derzeitiger Sicht vorteilhaften geometrischen Ausführung ist der Leiterzug bogenförmig auf einer entsprechend bogenförmigen Außenkante eines im wesentlichen L-förmigen Trägersubstrats ausgebildet.

Vorteile und Zweckmäßigkeiten der Erfindung ergeben sich im Übrigen aus der nachfolgenden Beschreibung eines Ausführungsbeispiels anhand der Figuren. Von diesen zeigen:
- Fig. 1: eine skizzenartige Darstellung eines ersten Ausführungsbeispiels der Erfindung,
- Fig. 2: eine skizzenartige Darstellung eines weiteren Ausführungsbeispiels der Erfindung,
- Fig. 3: eine skizzenartige Darstellung eines weiteren Ausführungsbeispiels der Erfindung, und
- Fig. 4: eine Draufsicht einer weiteren erfindungsgemäßen Antenne in einer speziellen geometrischen Konfiguration.

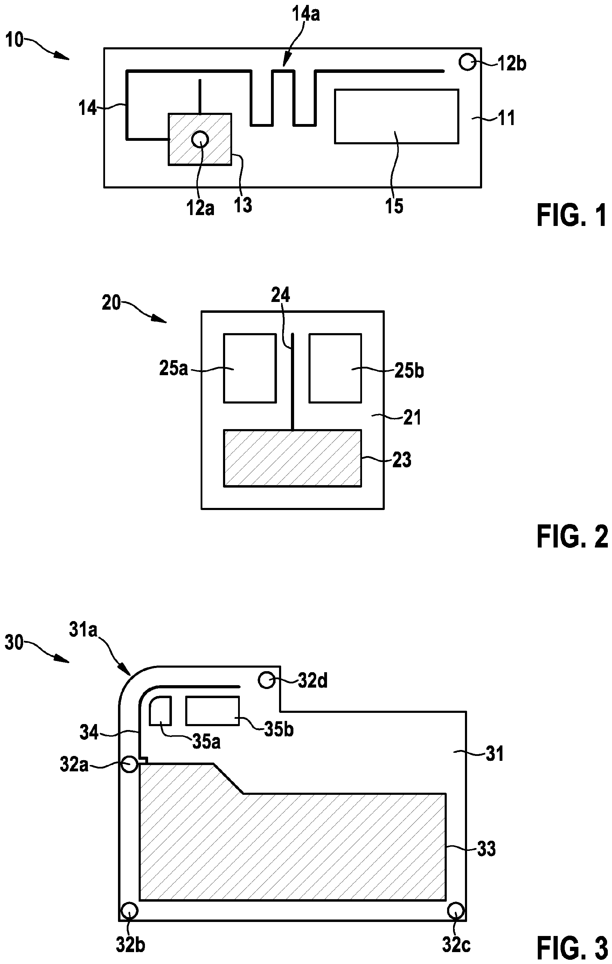

Fig. 1 zeigt schematisch eine Antenneneinheit 10, die beispielsweise in einem Programmiergerät eines Herzschrittmachers eingesetzt werden kann. Sie umfasst eine rechteckförmig konfigurierte Leiterplatte 11, die zur mechanischen Befestigung im Programmiergerät zwei Bohrungen 12a und 12b hat. Die Umgebung der Bohrung 12a ist als Anschlussbereich 13 flächig metallisiert, kann aber auch als Massefläche ausgeführt sein, während die Antennestruktur 14 über gedruckte Leiterstrukturen und ggf. über ein Anpassungsnetzwerk mit elektronischen Bauelementen zur Impedanzanpassung durch eine (nicht gezeigte) Antennenbuchse gespeist wird.

Von der Anschlussfläche 13 geht ein langgestreckter Leiterzug 14 aus, der die eigentliche Antenne bildet. Der Leiterzug 14 ist mehrfach abgewinkelt und hat einen mäanderförmig ausgestalteten Mittenbereich 14a, um insgesamt eine zur Erzielung einer gewünschten Impedanz benötigte Leiterzuglänge zu erreichen. Nahe dem freien Ende des Antennen-Leiterzuges 14 ist in der Leiterplatte 11 eine rechteckige Ausnehmung 15 vorgesehen, die zur Verringerung des Einflusses des Leiterplattenmaterials auf die Eigenschaften der Antenne 14 eingebracht ist.

Fig. 2 zeigt als weitere Ausführung eine Antennenbaugruppe 20, die auf einem quadratischen Keramik-Trägersubstrat 21 realisiert ist. Von einer Massefläche 23 geht hier eine relativ kurze, geradlinig gestreckte Antenne 24 aus, die wiederum als leitfähige Beschichtung auf dem Trägersubstrat 21 realisiert ist. Die Speisung der Antenne kann über Leiterstrukturen in der Massefläche 23 erfolgen, die auch Bauelemente zur Anpassung enthalten können. Beidseits der Antenne 24 sind Aussparungen 25a, 25b im Trägersubstrat zur Verbesserung der Eigenschaften der Antenne vorgesehen. Auch hier kann die Anschlussfläche 23 durch eine elektronische Baugruppe mit darüberhinausgehender Funktion ersetzt oder mit einer solchen kombiniert sein.

Fig. 3 zeigt als weiteres Ausführungsbeispiel eine Antennenbaugruppe 30, die grundsätzlich ähnlich aufgebaut ist wie die Antennenbaugruppen 10 und 20 nach Fig. 1 und 2, aber eine abweichende geometrische Konfiguration hat. Die Baugruppe umfasst hier ein weitgehend rechteckiges, aber an einer langen Seite mit einem Vorsprung 31a versehenes Trägersubstrat 31. Zudem ist eine Ecke des Trägersubstrats 31 im Bereich des Vorsprungs 31a abgerundet ausgeführt.

Dort sowie längs der Außenkante des Vorsprungs 31 a verläuft ein gleichermaßen bogenförmiger Leiterzug 34 als eigentliche Antenne der Baugruppe. Nahe dem Leiterzug sind hier wiederum zwei Ausnehmungen 35, 35b zur Verringerung des Einflusses des Leiterplattenmaterials auf die Eigenschaften der Antenne 34 vorgesehen. Außerdem hat das relativ große Trägersubstrat 31 insgesamt vier Befestigungsbohrungen 32a - 32d, von denen eine, nämlich die Bohrung 32a, nahe dem Verbindungspunkt der Antenne 34 einer Anschluss-Metallisierung 33 platziert ist.

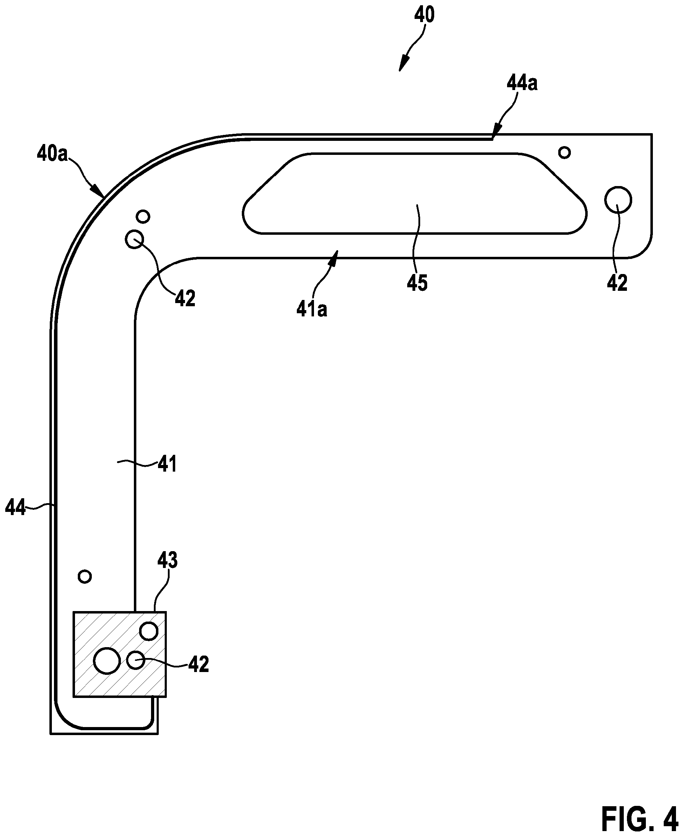

Fig. 4 zeigt als weiteres Ausführungsbeispiel eine im Wesentlichen L-förmig konfigurierte Antennenbaugruppe 40, deren Trägersubstrat 41 wieder aus einem herkömmlichen Leiterplattenmaterial besteht. Auch bei dieser Antennenbaugruppe ist ein Eckbereich 40a bogenförmig gestaltet, speziell im Verlauf eines Kreisbogens. Nahe dem unteren Ende des vertikalen Schenkels ist ein Antennen-Speisepunkt 43 vorgesehen. Hier befinden sich auch mehrere Befestigungs- und Justierbohrungen 42, die ebenso wie weitere Befestigungs- und Justierbohrungen an anderen Stellen der Antennenbaugruppe 40 mit Ziffer 42 bezeichnet sind.

Vom Antennen-Speisepunkt 43 geht auch hier ein langgestreckter und hier zweifach abgewinkelter Leiterzug 44 aus, der die eigentliche Antenne der Baugruppe bildet. Er verläuft nahe der bogenförmig gestalteten Außenkante des Trägersubstrats 41 und endet an einem Punkt 44a mit etwas Abstand vom freien Ende des zweiten (in der Figur horizontalen) Schenkels der "L". Im Endbereich der Antenne 44 ist wiederum eine Ausnehmung 45 im Trägersubstrat vorgesehen. Das unter der Ausnehmung stehenbleibende Trägerplattenmaterial dient als Stütz- bzw. Versteifungssteg 41a des Trägersubstrats 41.

Im Übrigen ist die Ausführung der Erfindung auch in einer Vielzahl von Abwandlungen der hier gezeigten Beispiele und weiter oben hervorgehobenen Aspekte der Erfindung möglich.

## Patentansprüche

1. Antenne (10, 20, 30, 40) eines Implantat-Programmiergerätes, welche insbesondere im 401-406 MHz-Band arbeitet, zur drahtlosen Kommunikation mit einem medizinelektronischen Implantat,
wobei die Antenne als Leiterzug (14, 24, 34, 44) auf einem steifen flächigen Trägersubstrat (11, 21, 31,41) ausgebildet ist, dessen Fläche um ein Vielfaches größer als die vom Leiterzug belegte Fläche ist.

2. Antenne (10, 20, 30, 40) nach Anspruch 1, wobei das Trägersubstrat (11, 21, 31,41) aus Leiterplattenmaterial besteht.

3. Antenne (10, 20, 30, 40) nach Anspruch 2, wobei das Trägersubstrat (11, 21, 31, 41) aus einem Epoxidharz-Glasfasergewebe-Verbundmaterial, insbesondere vom FR4-Typ, besteht.

4. Antenne (10, 20, 30, 40) nach Anspruch 2, wobei das Trägersubstrat (11, 21, 31,41) aus einem Material auf Hartpapierbasis, insbesondere vom FR2-Typ, besteht.

5. Antenne (10, 20, 30, 40) nach Anspruch 1 oder 2, wobei das Trägersubstrat (11, 21, 31,41) aus einer Keramik besteht, die insbesondere Aluminiumoxide, Aluminiumnitrit oder LTCC aufweist.

6. Antenne (10, 20, 30, 40) nach Anspruch 1 oder 2, wobei das Trägersubstrat (11, 21, 31,41) aus einem PTFE-Material besteht.

7. Antenne (10, 20, 30, 40) nach einem der vorangehenden Ansprüche, wobei benachbart zu einem Abschnitt des Leiterzuges (14, 24, 34, 44) mindestens eine Ausnehmung (15, 25a, 25b, 35a, 35b, 45) oder lokale Ausdünnung im Trägersubstrat (11, 21, 31,41) vorgesehen ist.

8. Antenne (10, 20, 30, 40) nach Anspruch 7, wobei die Ausnehmung (15, 25a, 25b, 35a, 35b, 45) oder lokale Ausdünnung nahe dem freien Ende des Leiterzuges (14, 24, 34, 44) vorgesehen ist.

9. Antenne (10, 20, 30, 40) nach einem der vorangehenden Ansprüche, mit Anpassungsmitteln zur Anpassung an eine Telemetrie-Sende-/Empfangsstufe oder deren Antennenanschluss, welche auf dem Trägersubstrat (11, 21, 31, 41) integriert sind.

10. Antenne (10, 20, 30, 40) nach einem der vorangehenden Ansprüche, wobei der Leiterzug (14, 24, 34, 44) bogenförmig auf einer entsprechend bogenförmigen Außenkante eines im Wesentlichen L-förmigen Trägersubstrats (11, 21, 31,41) ausgebildet ist.

11. Programmiergerät eines medizinelektronischen Implantats, insbesondere eines Herzschrittmachers, Kardioverters oder Neurostimulators, mit einer Antenne (10, 20, 30, 40) nach einem der vorangehenden Ansprüche.
